# EUROPÄISCHE PATENTSCHRIFT

(11) **EP 1 625 307 B1**
(45) Veröffentlichungstag und Bekanntmachung des Hinweises auf die Patenterteilung: **06.02.2008**
(21) Anmeldenummer: 04726102.9
(22) Anmeldetag: 07.04.2004
(51) Int. Cl.: F15B 13/04

(54) **VENTIL**
VALVE
SOUPAPE

(30) Priorität: 16.05.2003 DE 10323595
(43) Veröffentlichungstag der Anmeldung: 15.02.2006
(73) Patentinhaber: Hydac Fluidtechnik GmbH, 66280 Sulzbach/Saar (DE)
(72) Erfinder: BILL, Markus, 66606 St.Wendel (DE); BRUCK, Peter, 66484 Althornbach (DE); HOFFMANN, Alois, 66123 Saarbrücken (DE)
(74) Vertreter: Bartels, Martin Erich Arthur
(86) Internationale Anmeldenummer: PCT/EP2004/003698
(87) Internationale Veröffentlichungsnummer: WO 2004/102011

(56) Entgegenhaltungen:
- EP-A- 0 467 128
- EP-A- 0 503 188
- DE-A- 19 932 139
- GB-A- 901 061
- US-A- 4 848 721
- US-B1- 6 330 798
- PATENT ABSTRACTS OF JAPAN Bd. 2000, Nr. 04, 31. August 2000 (2000-08-31) & JP 2000 009250 A (MASUDA KENJI), 11. Januar 2000 (2000-01-11)

## Beschreibung

Die Erfindung betrifft ein Ventil, insbesondere Proportional-Sitzventil oder Schieberventil, mit einem Ventilgehäuse und mindestens drei, das Ventilgehäuse durchgreifenden Fluid-Anschlüssen sowie mit einem, im Ventilgehäuse geführten Hauptkolben und einem eine Vorsteuerung bewirkenden Vorsteuerkolben, der mittels einer bestrombaren Magneteinrichtung ansteuerbar ist gemäß der Merkmalsausgestaltung des Oberbegriffs des Patentanspruchs 1.

Bei einem bekannten Ventil nach der EP 0 893 607 A handelt es sich um ein magnetbetätigtes Ablaßventil, bei dem zwischen einem Lastdruckanschluß (P) und einem Ablaßanschluß (T) in einem Hubmodul eines Hubstaplers einem Hauptventilsitz ein Sitz-Schließelement zugeordnet ist, das in Schließrichtung von einem veränderbaren Unterschied zwischen dem Ablaßdruck und einem vom Lastdruck abgeleiteten Steuerdruck beaufschlagbar ist, wobei ein durch eine Magneteinrichtung betätigbares Vorsteuerventil mit Vorsteuerkolben für den Steuerdruck vorgesehen ist. Dem vom Hauptventilsitz und dem Sitz-Schließelement gebildeten Hauptventil ist eine Druckwaage mit Sitzventil-Dichtfunktion zugeordnet, die mit dem Hauptventil einen lastdruckunabhängigen Zweiwege-Stromregler bildet, der unter dem Lastdruck in Schließstellung des Hauptventils leckagefrei dicht ist.

Durch diese bekannte Lösung ist ein baulich einfaches, klein bauendes magnetbetätigtes Ablaßventil bekannt, mit dem es möglich ist, eine sog. Rampenfunktion lastdruckunabhängig zu realisieren. Unter Rampenfunktion versteht man dabei die Möglichkeit, die Durchflußmenge hubabhängig und druckunabhängig zu regeln. Es hat sich jedoch gezeigt, dass die bekannte Lösung für das Absenken der Last in hydraulischen Hubvorrichtungen nicht wie gewünscht die hohen Anforderungen erfüllt, nämlich bei geringer Leckage eine hohe Senkgeschwindigkeit ohne Last zu erreichen und die dahingehende Senkgeschwindigkeit feinfühlig zu dosieren.

Auf dem Markt sind ferner Steuereinrichtungen für hydraulisch arbeitende Hubeinrichtungen frei erhältlich, die direkt gesteuerte Ventile einsetzen, die jedoch bauartbedingt nicht für hohe Volumenströme geeignet sind, so dass in der Regel vorgesteuerten Ventilen der Vorzug gegeben wird. Bei sog. barometrisch vorgesteuerten Ventilen ist eine eigenständige Druckversorgung erforderlich, die den benötigten Druck für die Einstellung des Hauptkolbens bereit stellt. Dieser Druck beträgt in der Regel 10 bis 20 bar und wird oft von einer externen Versorgung, z.B. der Speisepumpe des Fahrantriebes, bei einem Gabelstapler mit Verbrennungsmotor erzeugt. Bei Staplern mit elektrischem Antrieb ist jedoch keine dahingehende externe Versorgung vorhanden, so dass der erforderliche Steuerdruck nur aus dem Lastdruck entnommen werden kann. Beim Senken ohne Last kann dann der zur Verfügung stehende Steuerdruck auf ca. 2 bar absinken mit der Folge, dass beim Senken-ohne-Last der Absenkvorgang behindert wird.

Durch die EP 0 503 188 A ist ein gattungsgemäßes Ventil ausgebildet als sog. bidirektionales Cartridge - Ventil bekannt mit einem Ventilgehäuse und drei das Ventilgehäuse durchgreifenden Fluidanschlüssen sowie mit einem, im Ventilgehäuse geführten Hauptkolben und einem eine Vorsteuerung bewirkenden Vorsteuerkolben, der mittels einer bestrombaren Magneteinrichtung ansteuerbar ist, wobei bei einer geöffneten Vorsteuerung Fluid von einem der beiden Anschlüsse, die von dem Hauptkolben ansteuerbar sind, über eine Querschnittsverengung im Hauptkolben und die Vorsteuerung zum dritten, durch den Vorsteuerkolben ansteuerbaren Anschluß gelangt, wobei aufgrund des damit einhergehenden Druckabfalls der Hauptkolben in jeweils eine, die beiden Fluidanschlüsse von der Fluidmenge her ansteuerbare Steuerposition gelangt, wobei zwischen Hauptkolben und Vorsteuerkolben eine Druckfeder angeordnet ist, wobei der Kolbenhub des Hauptkolbens bei geöffneter Vorsteuerung proportional dem Magnetstrom der Magneteinrichtung ist und wobei die Druckfeder in eine Ausnehmung des Hauptkolben eingreift; in die die Querschnittsverengung in Form einer Blende mündet. Es hat sich in der Praxis beim Einsatz dahingehender Ventile gezeigt, dass ein hemmfreier Betrieb beim Zusammenwirken von Vorsteuerkolben mit Hauptkolben nicht immer gewährleistet ist. Auch baut die bekannte Ventillösung kompliziert auf.

Ein solche Ventil ist in US 6 330 798 offenbart.

Ausgehend von diesem Stand der Technik liegt der Erfindung die Aufgabe zugrunde, ein Ventil zur Verfügung zu stellen, das in kostengünstiger Weise mit wenig Baukomponenten funktionssicher arbeitet. Eine dahingehende Aufgabe löst ein Ventil mit den Merkmalen des Patentanspruches 1 in seiner Gesamtheit.

Dadurch, dass gemäß dem kennzeichnenden Teil des Patentanspruches 1 an dem freien Ende der Druckfeder, das dem Vorsteuerkolben zugeordnet ist, ein Anlagestück angeordnet ist, das mit dem freien Ende des Vorsteuerkolbens über eine Anlagekugel in Verbindung steht, ist ein verbessertes Zusammenwirken zwischen Vorsteuerkolben und Hauptkolben geschaffen, insbesondere ein hemmfreier und mithin funktionssicherer Betrieb gewährleistet.

Es hat sich gezeigt, dass die erfindungsgemäße vorgesteuerte Proportional-Sitzventil- oder Schieberventillösung bei sehr niedrigem Vorsteuerdruck, beispielsweise < 2 bar, bereits voll öffnet und damit ein schnelles Absenken-ohne-Last ermöglicht.

Wird zum Öffnen der Vorsteuerung die Magneteinrichtung bestromt, wird der Hauptkolben aufgeschoben, wobei der Kolbenhub des Hauptkolbens proportional dem Magnetstrom ist. Da die Position des Hauptkolbens mithin immer der Magnetkraft entspricht, läßt sich dergestalt ein Ventil ausbilden, mit dem eine feinfühlige Dosierung der Senkgeschwindigkeit möglich ist bei gleichzeitig geringer Leckage für das erfindungsgemäße Ventil.

Bei einer bevorzugten Ausführungsform des erfindungsgemäßen Ventils ist zwischen Hauptkolben und Vorsteuerkolben eine Druckfeder angeordnet, wobei der Kolbenhub des Hauptkolbens bei geöffneter Vorsteuerung proportional dem Magnetstrom der Magneteinrichtung ist. Die am Hauptkolben angreifende Druckfeder meldet die Position des Hauptkolbens zurück an den Vorsteuerkolben und mithin an die Vorsteuerung, so dass etwaige Störgrößen, beispielsweise hervorgerufen durch Strömungskräfte, unmittelbar ausgeregelt werden können und die Stellung des Hauptkolbens entspricht mithin der aufgebrachten Magnetkraft. Bei unbestromter Magneteinrichtung kann das Ventil bedingt durch die Druckfeder von den beiden Anschlüssen ansteuerbar durch den Hauptkolben in der Art eines federbelasteten Rückschlagventils durchströmt werden.

Bei einer anderen Ausführungsform des erfindungsgemäßen Ventils ist vorzugsweise im Hauptkolben ein Wechselventil angeordnet, wobei das Wechselventil vorzugsweise die Querschnittsverengung aufweist. Bei der dahingehenden Ausbildung kann das Ventil unbestromt von einem Druckanschluß zum anderen sperren, die vom Hauptkolben ansteuerbar sind, und durch Bestromen der Magneteinrichtung bei entsprechenden Druckverhältnissen läßt sich dergestalt der Volumenstrom zwischen den beiden Fluid-Anschlüssen ansteuern. Bei einer alternativen Ausführungsform kann die Querschnittsverengung (Drossel oder Blende) auch in einem fluidführenden Kanal hinter dem Wechselventil in Richtung des Inneren des Hauptkolbens angeordnet sein.

Bei einer weiter bevorzugten Ausführungsform des erfindungsgemäßen Ventils weist die Magneteinrichtung mindestens einen Anker, eine Spule und ein Polrohr auf, die in der Art eines drückenden oder ziehenden Systems ausgebildet ist, d.h. dass der Anker beim Bestromen der Spule aus dem Polrohr sich heraus bzw. hinein bewegt, und dass bei Einsatz eines ziehenden Systems eine weitere Druckfeder den Vorsteuerkolben in Richtung einer geöffneten Vorsteuerung bewegt. Ist das "ziehende" Polrohr mit der genannten weiteren Druckfeder ausgestattet, die den Vorsteuerkolben in der offenen Position hält, was dem Zustand vollbestromt beim "drückenden" Polrohr entspricht, so kann durch Schalten der Magneteinrichtung die Vorsteuerung und damit das Ventil komplett geschlossen werden. Durch den Austausch eines "drückenden" Polrohres durch ein "ziehendes" Polrohr kann also aus dem stromlos geschlossenen Proportional-Sitzventil ein stromlos offenes Ventil gebildet werden. Sofern eine Vorsteuerfeder eine Einstellkraft auf den Vorsteuerkolben ausübt, ist dies im Hinblick auf die Schalteigenschaft des Magnetsystems nicht zwingend notwendig; sie verbessert aber das Zurückschalten des Vorsteuerkolbens und damit die Schaltdynamik für das Gesamtventil.

Bei einer weiteren bevorzugten Ausführungsform des erfindungsgemäßen Ventils ist die Vorsteuerung in der Art eines Schieberventils ausgebildet, bei dem ein zumindest an seinem freien Ende zylindrisch ausgebildeter Vorsteuerkolben in eine entsprechende Längsausnehmung in Teilen des Ventilgehäuses längsverfahrbar geführt ist. Hierdurch ist ein gleichmäßiges Schaltverhalten erreicht, und zwar unter verschiedensten Betriebsbedingungen, wobei durch Einhalten eines ausreichend geringen Dichtspaltes am Vorsteuerkolben die gewünschte Staplerdichtheit gewährleistet werden kann.

Bei einer anders gearteten Ausführungsform des erfindungsgemäßen Ventils ist vorzugsweise vorgesehen, dass die Vorsteuerung in der Art eines Sitzventils ausgebildet ist, bei dem am freien Ende des Vorsteuerkolbens ein bevorzugt kegelartiges Schließ- und Dichtteil angeordnet ist, das mit einem Sitzteil, gebildet von Teilen des Ventilgehäuses, zusammenwirkt. Bei der dahingehenden Ausführung als Sitzventil ist die Vorsteuerung leckagefrei, was allerdings den Nachteil mit sich bringt, dass der Vorsteuerkolben nicht mehr ideal druckausgeglichen und durch die Dichtung in seiner Bewegung auch reibungsbehaftet ist. Sofern die Vorsteuerung als Ventil ohne Dichtung ausgeführt ist, ist das Ventil nicht mehr leckagefrei, aber eine möglicherweise hemmende Reibung im Betrieb ist somit weitgehend ausgeschlossen, womit sichergestellt ist, dass das Ventil seine Drosselfunktion ausübt. Vorzugsweise können zur Erhöhung der Abdichtung am Außenumfang des Vorsteuerkolbens weitere Dichtteile angeordnet sein.

Das beschriebene Ventil ist speziell geeignet für alle Anwendungen, bei denen mit niedrigem Steuerdruck ein großer Volumenstrom anzusteuern ist, was häufig bei der Realisierung von Senkfunktionen bei Elektro-Staptern der Fall ist.
Das Proportional-Sitzventil kann generell als Proportional-Drosselventil für sehr große Volumenströme eingesetzt werden. Um das Δ_{P} bei hohen Volumenströmen gering zu halten, kann es erforderlich sein, den Sitz-Durchmesser im Ventilkörper zu vergrößern. Der notwendige Steuerdruck zum vollen Öffnen des Ventils steigt zwar dadurch an; liegt aber immer noch deutlich unter dem der bekannten barometrisch vorgesteuerten Ventile.

Bei einer bevorzugten Ausführungsform dient das erfindungsgemäße Ventil in einem Ventilsystem dazu, in Verbindung mit einer Druckwaage als verstellbare Meßblende eines Stromreglers eingesetzt zu werden. Bei der dahingehenden Ausgestaltung läßt sich die Durchflußmenge hubabhängig und druckunabhängig (Rampenfunktion) regeln und beim Absenken kann der zu beherrschende Volumenstrom von seinem Maximum her begrenzt werden, was der Erhöhung der Sicherheit dient.

Im folgenden wird die erfindungsgemäße Ventilkonstruktion in prinzipieller und nicht maßstäblicher Darstellung anhand der Zeichnung näher erläutert. Dabei zeigen in prinzipieller und nicht maßstäblicher Darstellung die
- Fig.1 und 2: einen Längsschnitt durch zwei verschiedene Ausführungsformen des erfindungsgemäßen Proportional-Sitzventiles, wobei in Blickrichtung auf die Figuren gesehen links oben das jeweilige Schaltsymbol des Ventils wiedergegeben ist;
- Fig.3: in der Art einer Schaltdarstellung den Einsatz des Ventils nach der Fig.1 bei einer Lastabsenkeinrichtung für Hubstaplereinrichtungen mit maximaler Volumenstrombegrenzung und mit Lastkompensation;
- Fig. 4: in vergrößerter Darstellung einen Längsschnitt durch den unteren Teil einer weiteren Ausführungsform in der Art eines Proportional-Schieberventiles einschließlich seines links oben wiedergegebenen Schaltsymbols.

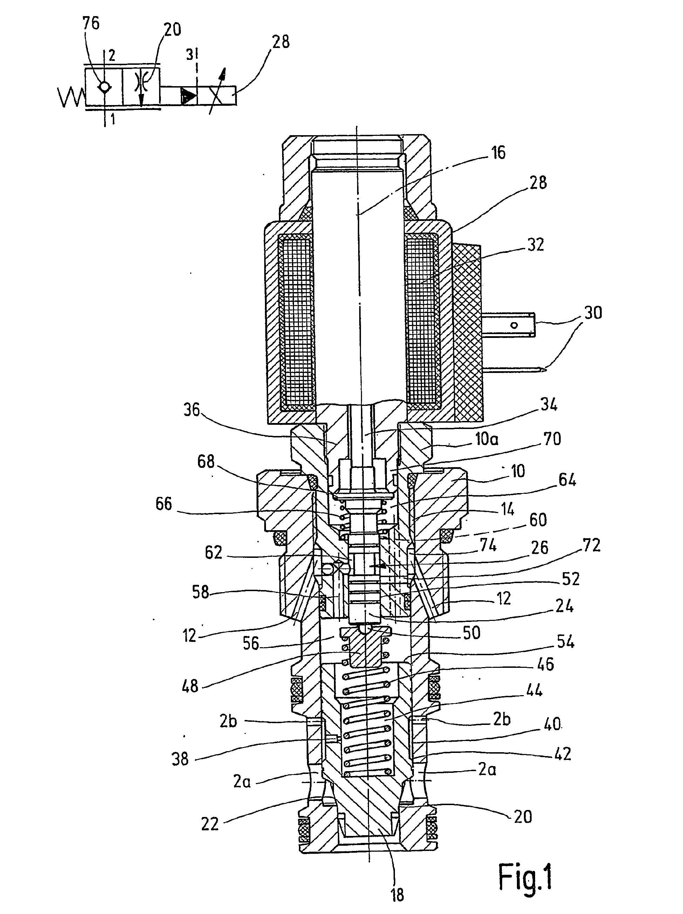

Das in der Fig.1 gezeigte, im Längsschnitt dargestellte Ventil stellt ein sog. Proportional-Sitzventil dar mit einem Ventilgehäuse 10, das außenumfangsseitig Dichtungen und Dichtpakete aufweist und das in der Art einer Einschraubpatrone ausgebildet ist zum Festlegen des Ventils an sonstigen Maschinen- oder Fahrzeugteilen zwecks Ansteuern eines hydraulischen Kreises (nicht dargestellt). Ferner kann das Ventil auch in der Art eines Einbausatzes ausgebildet sein. Das Ventilgehäuse 10 weist drei Fluidanschlüsse 1,2,3 auf, wobei der eine Fluidanschluß 1 stirnseitig am unteren Ende des Ventilgehäuses 10 eingreift und die beiden weiteren Anschlüsse 2 und 3 sind radial und außenumfangsseitig am Ventilgehäuse 10 angeordnet, wobei der Fluidanschluß 2 an zwei verschiedenen Stellen 2a, 2b das Ventilgehäuse 10 radial durchgreift und der dritte Fluidanschluß 3 mündet über Schrägbohrungen 12 in das Innere des Ventilgehäuses 10, das in diesem Bereich über einen mit einer Einschraubschräge 14 eingebrachten Ventileinsatz 10a verfügt. In dem Ventilgehäuse 10 ist axial zur Längsachse 16 des Ventils verfahrbar ein Hauptkolben 18 angeordnet, der an seinem freien Ende und benachbart gegenüberliegend zum Fluidanschluß 2a mit den zugeordneten Wandteilen des Ventilgehäuses ein Sitzventil 20 ausbildet, wobei hierfür der Hauptkolben 18 an seinem freien Ende mit einer konisch verlaufenden Ventilfläche 22 versehen ist. Neben dem Hauptkolben 18 ist innerhalb des Ventilgehäuses 10 ein Vorsteuerkolben 24 längsverfahrbar geführt, der Teil einer als Ganzes mit 26 bezeichneten Vorsteuerung ist.

In Blickrichtung auf die Fig.1 gesehen weist das Ventilgehäuse 10 an seinem oberen Ende eine bestrombare Magneteinrichtung 28 auf mit Anschlußsteckern 30 zum Anschluß an eine elektrische Einrichtung und zum Bestromen einer Spulenwicklung 32, die einen Anker 34 umfaßt, der innerhalb eines Profilrohres 36 längsverfahrbar gelagert ist und der Ansteuerung der Vorsteuerung 26 dient, insbesondere in Form des Vorsteuerkolbens 24. Der dahingehende Aufbau einer Magneteinrichtung 28 ist im Stand der Technik hinreichend bekannt, so dass an dieser Stelle hierauf nicht mehr näher eingegangen wird.

Gemäß Schaltdarstellung nach der Fig.1 befindet sich der Hauptkolben 18 in seiner geschlossenen Stellung, d.h. das Sitzventil 20 sperrt den Fluidweg zwischen den Fluid-Anschlüssen 1 und 2a ab. Eine radial am Außenumfang des Hauptkolbens 18 angeordnete Querschnittsverengung 38, vorzugsweise in Form einer Blende, mündet in eine Radialausnehmung 40 des Hauptkolbens 18 ein, die sich zwischen dem Fluidanschluß 2b erstreckt sowie einem radialen Überstand 42 des Hauptkolbens 18, der den Fluidanschluß 2a von der Radialausnehmung 40 trennt. Der Hauptkolben 18 ist mit einer Ausnehmung 44 versehen, in die die Blende 38 mündet und innerhalb dieser Ausnehmung 44, die sich in Richtung der Längsachse 16 erstreckt, ist eine Druckfeder 46 angeordnet, die mit ihrem einen freien Ende am Boden der Ausnehmung 44 anliegt und mit ihrem anderen freien Ende an einem Anlagestück 48 angreift, das an seinem freien Ende eine Anlagekugel 50 in einer entsprechenden Aussparung trägt, an deren Oberseite sich das freie Ende des Vorsteuerkolbens 24 abstützt. Auf diese Art und Weise ist ein hemmfreier Betrieb und Ansteuerung zwischen Vorsteuerkolben 24 und Hauptkolben 18 erreicht, auch bei etwaigen Verkantungsvorgängen, die durch die Anlagekugel 50 ausgleichbar sind.

Bei der gezeigten Lösung nach der Fig.1, die in Blickrichtung auf sie gesehen links oben von ihrer Funktion her in üblicher Schaltdarstellung wiedergegeben ist, bei der die dort gezeigten Fluidanschlüsse 1, 2 und 3 den Anschlüssen nach der Schnittdarstellung des Ventils entsprechen, ist die Vorsteuerung 26 in der Art eines Schieberventils realisiert, bei dem der zumindest an seinem freien Ende zylindrisch ausgebildete Vorsteuerkolben 24 in einer entsprechenden, im Querschnitt kreisförmigen Längsausnehmung 42 in Teilen des Ventilgehäuses 10 in Form des Ventileinsatzes 10a längsverfahrbar geführt ist. Der Vorsteuerkolben 24 ist außenumfangsseitig in üblicher Weise von Druckentlastungsrillen umgeben, die zumindest teilweise die Leckagedichtheit in diesem Bereich der Vorsteuerung 26 sicherstellen. Zwischen der Unterseite des Ventileinsatzes 10a und dem oberen Abschlußende des Hauptkolbens 18, das seine Rückseite 54 bildet, ist von der Innenumfangsseite des Ventilgehäuses 10 eine Steuerkammer 56 begrenzt, in die Längskanäle 58,60 des Ventileinsatzes 10a münden, wobei der eine Längskanal 58 mit seinem anderen Ende in eine Ringausnehmung 62 des Vorsteuerkolbens 24 mündet und der andere Längskanal 60 mündet mit seinem anderen freien Ende in eine Ringkammer 64, in der eine weitere Druckfeder 66 angeordnet ist, die sich mit ihrem einen unteren Ende an einem Innenumfang des Ventileinsatzes 10a abstützt und mit ihrem anderen Ende an einer Radialerweiterung 68 des Vorsteuerkolbens 24, wobei in der gezeigten Schaltdarstellung nach der Fig.1 die Radialausnehmung 68 sich mit ihrem Außenflansch am vorderen Ende des Magnetgehäuses 70 abstützt, das an dieser Stelle über eine Einschraubstrecke in den Ventileinsatz 10a eingebracht ist. Des weiteren mündet ein radialer Ringkanal 72 in eine Radialkammer 74 zwischen Innenumfangsseite des oberen Endes des Ventilgehäuses 10 und der Außenumfangsseite des Ventileinsatzes 10a in diesem Bereich. In diese Radialkammer 74 mündet wiederum der Fluidanschluß 3 und am gegenüberliegenden Ende ist in der gezeigten Schaltstellung nach der Fig.1 der Ringkanal 72 vom Außenumfang des Vorsteuerkolbens 24 verschlossen, wobei bei betätigtem Vorsteuerkolben 24, bei dem dieser in Blickrichtung auf die Fig.1 gesehen, durch die Magneteinrichtung 28 nach unten gedrückt ist, eine fluidführende Verbindung herstellbar ist zwischen Steuerkammer 56, Längskanal 58, Ringausnehmung 62, Ringkanal 72, Radialkammer 74 und dem Fluidanschluß 3 über die kanalartigen Schrägbohrungen 12.

Des besseren Verständnisses wegen wird nunmehr das in Fig.1 dargestellte Proportional-Sitzventil, insbesondere vorgesehen für den Einsatz bei hydraulisch arbeitenden Hubeinrichtungen, anhand eines Arbeitsbeispiels näher beschrieben. Wird die Magneteinrichtung 28 über die Anschlußstekker 30 bestromt, wandert der Anker 34 unter der Feldwirkung der Spulenwicklung 32 aus dem Polrohr 36 hinaus und betätigt dergestalt den Vorsteuerkolben 24 der Vorsteuerung 26 entgegen der Wirkung der weiteren Druckfeder 66, die mit ihrer Rückstellkraft die Tendenz hat, die Radialerweiterung 68 in Anlage zu halten mit dem unteren Ende des Magnetgehäuses 70. Die genannte Magnetkraft reicht aber jedenfalls aus, entgegen der Wirkung der weiteren Druckfeder 66 die Vorsteuerung 26 zu öffnen und Vorsteueröl vom Lastanschluß 2 strömt über die jeweilige Anschlußstelle 2b in die Radialausnehmung 40 des Hauptkolbens 18 und von dort über die Querschnittsverringerung 38 (Blende) in die Ausnehmung 44 des Hauptkolbens 18, in dem die Druckfeder 46 angeordnet ist. Von dort strömt das Vorsteueröl in die Steuerkammer 56 und von dort aus über den Längskanal 58, die Ringausnehmung 62 im Vorsteuerkolben 24 in den Ringkanal 72 und von dort aus über die Radialkammer 74 über die Schrägbohrungen 12 zum Fluidanschluß 3. Hierbei kommt es zum Druckabfall auf der Rückseite 54 des Hauptkolbens 18 und durch den auf die Kreisringfläche zwischen Kolbenaußendurchmesser und Ventilsitzdurchmesser des Hauptkolbens 18 an der Stelle seines Sitzventils 20 wirkenden Lastdruckes wird der Hauptkolben 18 gegen die Wirkung der Druckfeder 46 in Blickrichtung auf die Fig.1 gesehen nach oben aufgeschoben. Der dahingehende Kolbenhub des Hauptkolbens 18 ist proportional dem Magnetstrom. Die im Hauptkolben 18 befindliche Druckfeder 46 meldet die Position des Hauptkolbens 18 zurück auf den Vorsteuerkolben 24, so dass Störgrößen, wie z.B. Strömungskräfte, dergestalt ausgeregelt werden können und die Position des Hauptkolbens 18 entspricht somit immer der Magnetkraft der Magneteinrichtung 28 im bestromten Zustand. Unbeströmt nimmt der Hauptkolben 18 seine in der Fig.1 gezeigte Stellung ein und in dieser Stellung wirkt das Ventil aufgrund der Druckfeder 46 wie ein federbelastetes Rückschlagventil 76 bezogen auf die Ansteuerung des möglichen Fluidstroms zwischen den Fluid-Anschlüssen 1 und 2.

Mit der dahingehenden Anordnung ist ein vorgesteuertes Proportional-Sitzventil realisiert, das bei sehr niedrigem Vorsteuerdruck (beispielsweise <2 bar) bereits voll öffnet und damit ein schnelles Senken-ohne-Last ermöglicht, so dass sein Einsatz insbesondere bei elektrobetriebenen Hubstaplern interessant ist, die über keine externe Versorgung verfügen, die notwendig ist, um den benötigten Druck für die Einstellung des Hauptkolbens bei barometrisch vorgesteuerten Ventilen, wie sie im Stand der Technik bekannt sind, sicherzustellen.

Die Vorsteuerfeder in Form der weiteren Druckfeder 66 ist nicht unbedingt erforderlich, sie verbessert aber, wie bereits dargelegt, das Zurückschalten des Vorsteuerkolbens 24 und damit die Dynamik des Ventils als Ganzes. Die in Fig.1 realisierte Vorsteuerung 26 ist in der Art eines Schieberventils ausgebildet, wobei dies die beste Lösung ist für das gleichmäßige Schaltverhalten unter verschiedenen Betriebsbedingungen, was jedoch mit dem Nachteil einhergeht, dass das in Fig.1 dargestellte Ventil entsprechend leckagebehaftet ist. Durch Einhalten eines ausreichend geringen Dichtspaltes am Vorsteuerkolben 24 kann jedoch die gewünschte Staplerdichtheit gewährleistet werden.

Das in der Fig.1 verwendete Polrohr 26 ist in der Art eines sog. drückenden Systems ausgebildet, bei dem der Anker 34 beim Bestromen der Spulenwicklung 32 aus dem Polrohr 36 austritt. Bei "ziehenden" Systemen, also bei einem sog. "ziehenden" Polrohr, bewegt sich jedoch der Anker 34 in das Polrohr 36 hinein. Ist das "ziehende" Polrohr mit einer Druckfeder (nicht dargestellt) ausgestattet, die den Vorsteuerkolben 24 in der offenen Position hält, was dem Zustand vollbestromt beim drückenden Polrohr 36 entspricht, so kann durch Schalten der Magneteinrichtung 28 die Vorsteuerung 26 und damit das Ventil komplett geschlossen werden. Durch den Austausch eines "drückenden" Polrohres 36 durch ein "ziehendes" Polrohr kann also aus dem stromlos geschlossenen Proportional-Sitzventil in einfacher Weise ein stromlos offenes Ventil gemacht werden, sofern die praktischen Einsatzbelange dies notwendig erscheinen lassen.

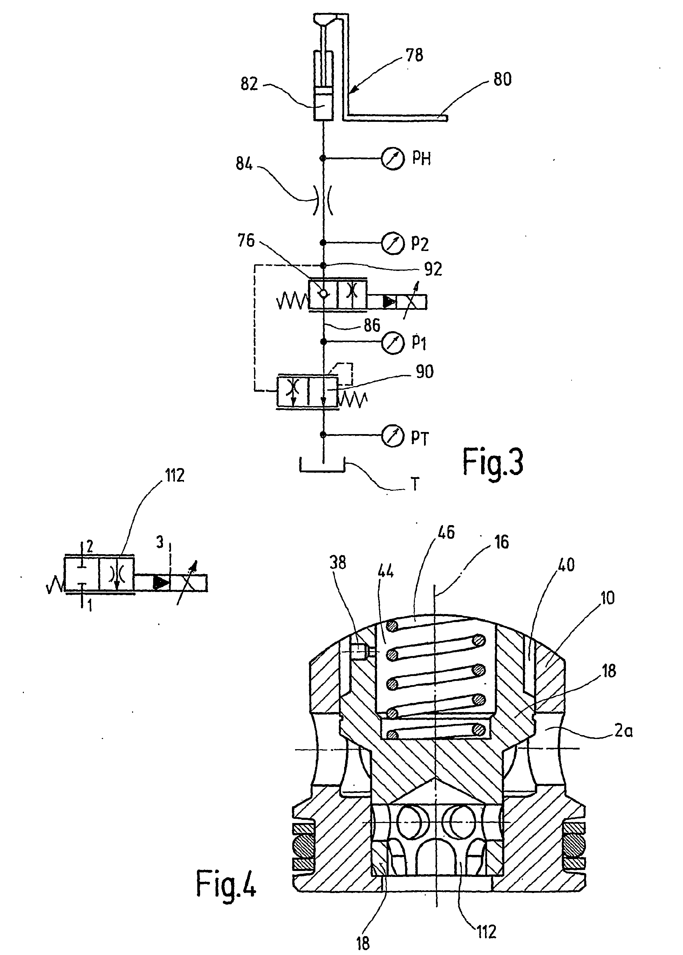

In der Fig.3 ist ein Anwendungsbeispiel des in der Fig.1 gezeigten Proportional-Sitzventils gezeigt für eine als Ganzes mit 78 bezeichnete, hydraulisch arbeitende Hubeinrichtung. Die hydraulische Hubeinrichtung 78 weist eine Lastgabel 80 üblicher Bauart auf, die über einen Arbeitszylinder 82 heb- und senkbar ist. Das Verhalten des Hubgerüstes der Hubeinrichtung 78 ist der einfacheren Betrachtung wegen von seinem hydraulischen Verhalten her als Drossel 84 wiedergegeben. Im übrigen ist die Kolbenseite des Arbeitszylinders 82 über eine Anschlußleitung 86 an den Tank T anschließbar. Die symbolisch dargestellten Manometer mit den Bezeichnungen P_{H}, P₂, P₁ und P_{T} würden im Rahmen eines Versuchsaufbaues den Abgriff von Druckwerten in einzelnen Verfahrstellungen der Hubeinrichtung 78 innerhalb der Anschlußleitung 86 ermöglichen. Wie die Darstellung nach der Fig.3 des weiteren zeigt, ist in die Anschlußleitung 86 eine an sich bekannte Druckwaage 90 mit Drosselfunktion angeschlossen und diese wird von dem anstehenden Druck in der Anschlußleitung 86 über die Anschlußstelle 92 angesteuert. Dergestalt ist gemäß der Darstellung nach der Fig.3 ein Ventilsystem realisiert, bestehend aus dem Ventil nach der Fig.1 und in Verbindung mit der an sich bekannten Druckwaage 90 ist eine einstellbare Meßblende eines Stromreglers realisiert. Das Proportional-Sitzventil nach der Fig.1 ist dergestalt als Proportional-Drossel-Ventil für sehr große Volumenströme einsetzbar und mit der gezeigten Ventilsystemlösung nach der Fig.3 läßt sich der maximale Volumenstrom beim Absenken der Lastgabel 80 (mit oder ohne Last) begrenzen, was der Sicherheit beim Betrieb der Hubeinrichtung zugute kommt. Insbesondere kann mit der vorliegenden Lösung bei niedrigem Steuerdruck ein großer Volumenstrom angesteuert werden.

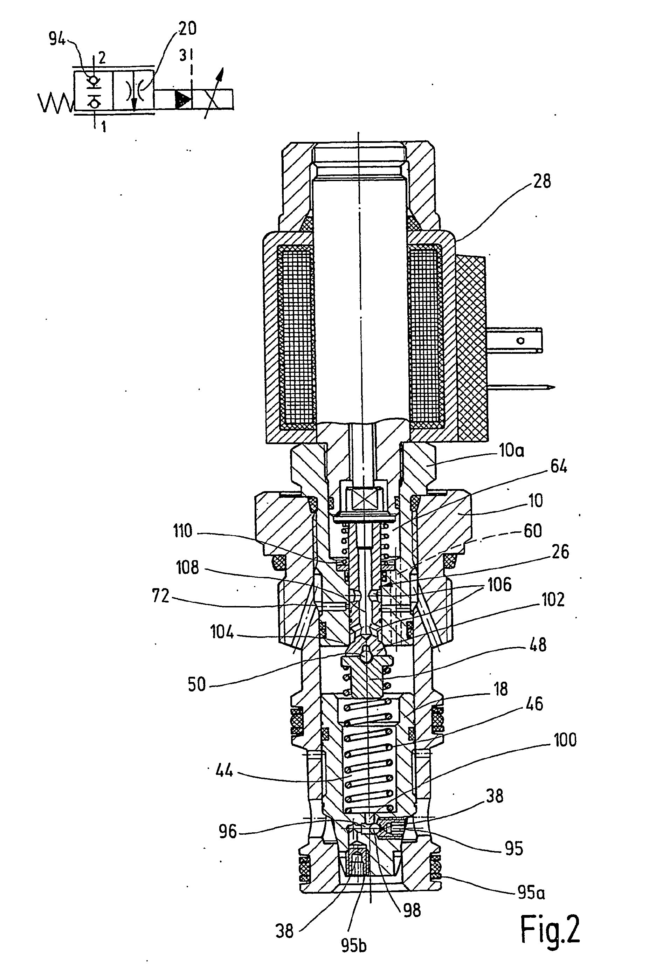

Die Ausführungsform nach der Fig.2 stellt eine Bauvariante dar zu der Ausführungsform nach der Fig.1 und wird demgemäß nur noch insofern erläutert, als sie sich wesentlich von der Ausführungsform nach der Fig.1 unterscheidet. Insofern werden auch für dieselben Teile dieselben Bezugszeichen verwendet, wie in der Fig.1, und das bisher Gesagte gilt dann auch insoweit für die geänderte Ausführungsform nach der Fig.2.

Bei der Ausführungsform nach der Fig.2 ist am unteren stirnseitigen Ende des Hauptkolbens 18 ein Wechselventil 95 angeordnet, wobei das Wechselventil 95 die Querschnittsverengung aufweist. Die dahingehende Blendenfunktion ist zweimal in beiden Durchströmrichtungen von 1 nach 2 und umgekehrt bezogen auf die Fluidanschlüsse vorhanden. Das Wechselventil 95 weist eine in einem Querkanal 96 bewegbare Ventilkugel 98 auf, die in Abhängigkeit der Fluidanströmrichtung von Fluidanschluß 1 nach 2 oder umgekehrt einmal die Fluidanschlußstelle des einen Wechselventileinsatzes 95a bzw. des anderen Wechselventileinsatzes 95b mit ihrer jeweiligen Querschnittsverengung 38 sperrt. Der genannte Querkanal 96 weist in Längsrichtung des Ventils einen Längskanal 100 auf, der in die Ausnehmung 44 im Hauptkolben 18 mit der Druckfeder 46 mündet. Bei der Ausführungsform nach der Fig.2 ist die Vorsteuerung 26 als Sitzventil mit Dichtung ausgebildet. Hierzu weist der Vorsteuerkolben 24 an seinem unteren freien Ende ein kegelartiges Schließ- und Dichtteil 102 auf, das mit einem Sitzteil 104 am unteren Ende des Ventileinsatzes 10a zusammenwirkt. Anstelle des Längskanals 58 weist die modifizierte Lösung nach der Fig.2 im Vorsteuerkolben 24 Querkanäle 106 auf, die im übrigen über einen mittigen Längskanal 108 fluidführend miteinander verbunden sind, so dass dergestalt bei geöffneter Vorsteuerung 26 der Fluidfluß von Fluidanschluß 2 zu Fluidanschluß 3 gewährleistet ist. Des weiteren weist der Vorsteuerkolben 24 außenumfangsseitig ein Dichtsystem 110 innerhalb der Ringkammer 64 auf. Bei der gezeigten Ausführung als Sitzventil nach der Fig.2 ist auch die Vorsteuerung 26 leckagefrei, wobei der Vorsteuerkolben 24 nicht mehr ideal druckausgeglichen, sondern vielmehr durch das Dichtsystem 110 auch reibungsbehaftet ausgeführt ist. Würde man auf die genannte Dichtung verzichten, würde sich der Nachteil der Reibungsbehaftung nicht einstellen. Das genannte Ventil wäre dann aber nicht mehr leckagefrei.

Mit dem genannten Ventil nach den Fig.1 und 2 lassen sich hohe Senkgeschwindigkeiten ohne Last bei hydraulischen Hubeinrichtungen erreichen, bei gleichzeitig feinfühliger Dosierung der Senkgeschwindigkeit und bei geringer Leckage.

Die Fig. 4 betrifft eine geänderte Ventilausführungsform gegenüber den vorgestellten Varianten nach den Fig. 1 und 2. So betrifft die Fig. 4 den unteren Ventilteil, der in der Art eines Schieberventiles insbesondere Proportional-Schieberventiles ausgebildet ist. Anstelle des bisher beschriebenen konischen Ventilsitzes 20 ist das freie Ende des Hauptkolbens 18 zylindrisch ausgebildet, und ist in einer zylindrischen Innenumfangsfläche des unteren Endes des Ventilgehäuses 10 geführt. Bei angehobenem Hauptkolben 18 wird dergestalt über das Fluidführungsteil 112 die fluidführende angedrosselte Verbindung zwischen dem Ventilanschluß 2a und der freien Fluideintrittseite am stirnseitigen Ende des Ventilgehäuses 10 hergestellt. Die entsprechende Schaltdarstellung ist in Blickrichtung auf die Fig. 4 gesehen links oben wiedergegeben, wobei die Vorsteuerung für diese als Schieberventil ausgebildete Ventilvariante entsprechend ausgebildet ist, wie vorstehend beschrieben für die Ventilvarianten nach den Fig. 1 und 2.

## Patentansprüche

1. Ventil, insbesondere Proportional-Sitzventil oder Schieberventil, mit einem Ventilgehäuse (10) und mindestens drei, das Ventilgehäuse durchgreifenden Fluid-Anschlüssen (1,2,3) sowie mit einem, im Ventilgehäuse (10) geführten Hauptkolben (18) und einem eine Vorsteuerung (26) bewirkenden Vorsteuerkolben (24), der mittels einer bestrombaren Magneteinrichtung (28) ansteuerbar ist, wobei bei einer geöffneten Vorsteuerung (26) Fluid von einem (2) der beiden Anschlüsse (1,2), die von dem Hauptkolben (18) ansteuerbar sind, über eine Querschnittsverengung (38) im Hauptkolben (18) und die Vorsteuerung (26) zum dritten, durch den Vorsteuerkolben (24) ansteuerbaren Anschluß (3) gelangt, wobei aufgrund des damit einhergehenden Druckabfalls der Hauptkolben (18) in jeweils eine, die beiden Fluidanschlüsse (1,2) von der Fluidmenge her ansteuerbare Steuerposition gelangt, wobei zwischen Hauptkolben (18) und Vorsteuerkolben (24) eine Druckfeder (46) angeordnet ist, wobei der Kolbenhub des Hauptkolbens (18) bei geöffneter Vorsteuerung (26) proportional dem Magnetstrom der Magneteinrichtung (28) ist und wobei die Druckfeder (46) in eine Ausnehmung (44) des Hauptkolbens (18) eingreift, in die die Querschnittsverengung (38) in Form einer Blende mündet, **dadurch gekennzeichnet, dass** an dem freien Ende der Druckfeder (46), das dem Vorsteuerkolben (24) zugeordnet ist, ein Anlagestück (48) angeordnet ist, das mit dem freien Ende des Vorsteuerkolbens (24) über eine Anlagekugel (50) in Verbindung steht.

2. Ventil nach Anspruch 1, **dadurch gekennzeichnet, dass** im Hauptkolben (18) ein Wechselventil (94) angeordnet ist, und dass das Wechselventil (94) die Querschnittsverengung (38) aufweist.

3. Ventil nach Anspruch 1 oder 2, **dadurch gekennzeichnet, dass** die Magneteinrichtung (28) mindestens einen Anker (34), eine Spule (32) und ein Polrohr (36) aufweist und in der Art eines drückenden oder ziehenden Systems ausgebildet ist, d.h. dass der Anker (34) beim Bestromen der Spule (32) aus dem Polrohr (36) sich heraus bzw. hinein bewegt, und dass bei Einsatz eines ziehenden Systems eine weitere Druckfeder den Vorsteuerkolben (24) in Richtung einer geöffneten Vorsteuerung (26) bewegt.

4. Ventil nach einem der Ansprüche 1 bis 3, **dadurch gekennzeichnet, dass** die Vorsteuerung (26) in der Art eines Schieberventils ausgebildet ist, bei dem ein zumindest an seinem freien Ende zylindrisch ausgebildeter Vorsteuerkolben (24) in eine entsprechende Längsausnehmung (52) in Teilen (10a) des Ventilgehäuses (10) längsverfahrbar geführt ist.

5. Ventil nach einem der Ansprüche 1 bis 4, **dadurch gekennzeichnet, dass** die Vorsteuerung (26) in der Art eines Sitzventils ausgebildet ist, bei dem am freien Ende des Vorsteuerkolbens (24) ein Schließ- und Dichtteil (102) angeordnet ist, das mit einem Sitzteil (104), gebildet von Teilen (10a) des Ventilgehäuses (10), zusammenwirkt.

6. Ventil nach einem der Ansprüche 1 bis 5, **dadurch gekennzeichnet, dass** am Außenumfang des Vorsteuerkolbens (24) weitere Dichtteile in Form eines Dichtsystems (110) angeordnet sind.

7. Ventilsystem, bestehend aus einem Ventil mit den Merkmalen eines der Patentansprüche 1 bis 6, das in Verbindung mit einer an sich bekannten Druckwaage (90) als einstellbare Meßblende eines Stromreglers einsetzbar ist.

## Claims

1. Valve, in particular a proportional seat valve or spool valve, with a valve housing (10) and at least three fluid connections (1, 2, 3) engaging the valve housing as well as a main piston (18) located in the valve housing (10), and a pre-control piston (24) acting on a pre-controller (26), the same being controllable by means of a powered magnet means (28), whereby fluid flows from one (2) of the two connections (1, 2) that can be controlled by the main piston (18) via a cross-sectional narrowing (38) in the main piston (18) when the pre-controller (26) is open, and from the pre-controller (26) to the third connection (3) that can be controlled by means of the pre-control piston (24), whereby the main piston (18) is moved into a control position adjusting the two fluid connections (1, 2) according to the fluid quantity due to the simultaneously occurring pressure loss, whereby a pressure spring (46) is located between the main piston (18) and the pre-control piston (24), and whereby the piston stroke of the main piston (18) is proportional to the magnetic flow of the magnet means (28) when the pre-controller (26) is open, and whereby the pressure spring (46) engages a recess (44) of the main piston (18) into which the cross-sectional narrowing (38) opens in the way of a baffle, **characterised in that** an abutment piece (48) is located at the free end of the pressure spring (46) that is associated with the pre-control piston (24), which is connected with the free end of the pre-control piston (24) via an abutment sphere (50).

2. Valve according to Claim 1, **characterised in that** an alternating valve (94) is located in the main piston (18), and **in that** the alternating valve (94) comprises the cross-sectional narrowing (38).

3. Valve according to Claim 1 or 2, **characterised in that** the magnet means (28) comprises at least one anchor (34), one coil (32) and one pole pipe (36), and is designed in the way of a pushing or pulling system, e.g. **in that** the anchor (34) is moved out of or into the pole pipe (36) when the coil (32) is powered, and **in that** a further pressure spring of the pre-control piston (24) is moved in the direction of an open pre-controller (26) when a pulling system is used.

4. Valve according to one of the Claims 1 to 3, **characterised in that** the pre-controller (26) is formed in the way of a spool valve, where a pre-control piston (24) formed cylindrically at least at its free end is held longitudinally displaceable within a corresponding longitudinal recess (52) in parts (10a) of the valve housing (10).

5. Valve according to one of the Claims 1 to 4, **characterised in that** the pre-controller (26) is designed in the way of a seat valve, where a closure and sealing part (102) is located at the free end of the pre-control piston (24) and co-operates with a seat part (104) formed by parts (10a) of the valve housing (10).

6. Valve according to one of the Claims 1 to 5, **characterised in that** further sealing parts in the form of a sealing system (110) are located on the outer circumference of the pre-control piston (24).

7. Valve system, consisting of a valve with the characteristics described in one of the Claims 1 to 6, that can be used as an adjustable measuring baffle of a flow controller in connection with known pressure scales (90).

## Revendications

1. Soupape, en particulier soupape proportionnelle à siège ou soupape à coulisse, comportant une cage de soupape (10) et au moins trois raccords de fluide (1, 2, 3) qui traversent la cage de soupape, comportant en outre un piston principal (18) guidé dans la cage de soupape (10) et un piston pilote (24) qui déclenche une commande pilote (26) et qui peut être commandé à l'aide d'un dispositif magnétique (28) susceptible d'être mis sous tension, dans laquelle soupape
- quand une commande pilote (26) est ouverte, du fluide provenant d'un (2) des deux raccords (1, 2) susceptibles d'être commandés par le piston principal (18) parvient par l'intermédiaire d'un rétrécissement de section (38) dans le piston principal (18) et de la commande pilote (26) jusqu'au troisième raccord (3) susceptible d'être commandé par le piston pilote (24),
- du fait de la baisse de pression qui en résulte, le piston principal (18) prend une position de commande susceptible de commander les deux raccords de fluide (1, 2) quant à la quantité de fluide,
- un ressort de pression (46) est disposé entre le piston principal (18) et le piston pilote (24),
- quand la commande pilote (26) est ouverte, la course du piston principal (18) est proportionnelle au courant magnétique du dispositif magnétique (28) et
- le ressort de pression (46) pénètre dans une cavité (44) du piston principal (18), dans laquelle débouche le rétrécissement de section (38) sous forme d'un diaphragme,
**caractérisée en ce qu'**une pièce de contact (48), qui est en liaison avec l'extrémité libre du piston pilote (24) par l'intermédiaire d'une sphère de contact (50) se trouve à l'extrémité libre du ressort de pression (46) associé au piston pilote (24).

2. Soupape selon la revendication 1, **caractérisée en ce qu'**une soupape de sélection (94) est disposée dans le piston principal (18) et que la soupape de sélection (94) présente le rétrécissement de section (38).

3. Soupape selon la revendication 1 ou 2, **caractérisée en ce que** le dispositif magnétique (28) comporte au moins un induit (34), une bobine (32) et un tube polaire (36) et est conçu à la manière d'un système de poussée ou de traction, c'est-à-dire que l'induit (34) sort du tube polaire (36) ou y entre quand la bobine (32) est sous tension et que, quand on utilise un système de traction, un autre ressort de pression déplace le piston pilote (24) en direction d'une commande pilote (26) ouverte.

4. Soupape selon l'une des revendications 1 à 3, **caractérisée en ce que** la commande pilote (26) est conçue à la manière d'une soupape à coulisse, dans laquelle un piston pilote (24), cylindrique au moins à son extrémité libre, est guidé pour se déplacer longitudinalement dans une cavité oblongue (52) correspondante située dans des parties (10a) de la cage de soupape (10).

5. Soupape selon l'une des revendications 1 à 4, **caractérisée en ce que** la commande pilote (26) est conçue à la manière d'une soupape à siège, dans laquelle une pièce de fermeture et d'étanchéité (102) qui coopère avec une partie de siège (104) formée par des parties (10a) de la cage de soupape (10), est disposée à l'extrémité libre du piston pilote (24).

6. Soupape selon l'une des revendications 1 à 5, **caractérisée en ce que** d'autres pièces d'étanchéité sous forme d'un système d'étanchéité (110) sont disposées sur le pourtour extérieur du piston pilote (24).

7. Système de soupape, composé d'une soupape ayant les caractéristiques d'une des revendications 1 à 6, qui peut être utilisé comme diaphragme de mesure réglable d'un régulateur de courant en liaison avec un manomètre à piston (90) connue en soi.
